# EUROPEAN PATENT APPLICATION

(11) **EP 2 457 545 A1**
(43) Date of publication of application: **30.05.2012**
(21) Application number: 11425266.1
(22) Date of filing: 31.10.2011
(51) Int. Cl.: A61F 5/02

(54) **A device provided with a sensor that activates an alarm buzzer which induces the user to keep a correct postural position**

(30) Priority: 29.11.2010 IT NA20100057
(71) Applicant: Di Bonito, Antonio, 80078 Pozzuoli (IT)
(72) Inventor: Di Bonito, Antonio, 80078 Pozzuoli (IT)

(57) **Abstract**

The purpose of this invention is aimed to correct the paramorphism that grow in the sagittal plane, in which the subject is not able to keep a proper posture in the space.

Nowadays, the corrective action to adjust the postural disequilibrium is the kinesitherapy activity.

The object of this patent concerns a device that performs proprioceptive actions by stimulating elements, due to activation of one (1) or both alarm sensors (2), that force the subject to keep a set of structural alignments that induce the same to a vigilant presence on its position in space, as a conditioned reaction. Table 1, Fig 1.

## Description

People from their birth are always in conflict with the force of gravity.

The opposition of the body muscles against the force of gravity begins in the early moments of life, when the child moves from the quadrupedal position to reach a standing position.

The bone structure, to maintain and sustain the body in space, must be maintained in a proper position continuously through a specific neuro-muscular balance.

The exact muscle tension and muscle balance must obtain the correct perpendicularity of the forces that gravitate on the bone surfaces.

This is important because, as contended by Delpech's theory, if a bone suffers an homogeneous pressure on its surface, it grows uniformly and entirely; otherwise, if the bone suffers a lower pressure in certain points, in these points it grows less than the others.

This means that the incorrect body behavior, if kept over time, may cause alteration of the force loads on the bone joints; this can produce a postural disequilibrium.

Therefore, it's necessary to take care and adjust the body posture by the first years of life, as it can turn in a subsequent state of dimorphism.

The purpose of this invention is aimed to correct these paramorphism that grow in the sagittal plane, in which the subject is not able to keep a proper posture in the space.

Nowadays, the corrective action to adjust the postural disequilibrium is the kinesitherapy activity. The results of postural adjustment are obtained by a proprioception therapy, in which the patient is subjected to stimuli to reach the correct posture, induced by a therapist and checked in a squared mirror.

With usual therapy, the results obtained lasts the time duration of the activity in the gym. Then, after gym activity, they return their "dominant" postural position, in which the patient has structured its body schema.

It is proved that the short time that people dedicate to rehabilitation is infinitesimally small compared to the postural improper action that they assume in their relation life.

Thus, based on what we said, we need to create in those subjects a "stimulation alert", so that the person can dominate and check the posture during every moment of its ordinary life.

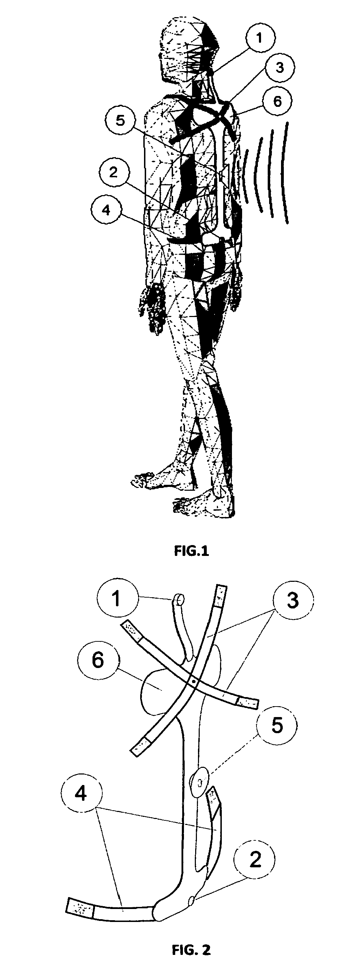

The object of this patent concerns a device that performs proprioceptive actions by stimulating elements, that force the subject to keep a set of structural alignments that induce the person to a vigilant presence on its position in space, as a conditioned reaction. Table 1, Fig 1.

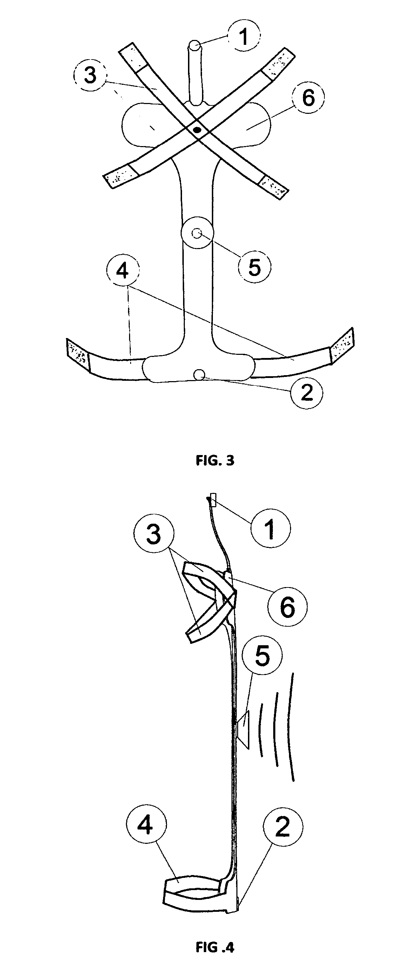

The device is characterized by a celtic cross shape, Table 2, Fig 3. It is positioned on the patient's back, Table 1, Fig 1.

At the extremities it consists of two bands (3) that surround, from back to front, Table 1, Fig 2, the humeral girdle; as this device pulls back the shoulders, it produces an adduction of the shoulder, Table 1, Fig.1.

This is a static mechanism, maintained by the resistance of the bands (3), which keep the shoulders joined to the rigid structure (6) Table 1, Fig.1.

The upper part of the cross, in the neck area, is characterized by a curve on the sagittal plane, which is calculated on the physiological curvature relative to the users, Table 2, Fig 4, Table 1, Fig.1.

In the middle of the curve there is a sensor (1) that is the proprioceptive element to which the subject must refer to obtain the correct position of the head, Table 1, Figure 1. In practice, the displacement of the head from the sensor (1) activates a sound (5) that pushes the subject to relocate its posture in a correct way, Table 1, Fig 1.

In the lower part, in lumbar region, there is another curve and at its top there is another sensor (2) Table 1, Figure 1, Table 2, Fig 3, 4. Also this curve is calculated in relation to the age and characteristics of the user.

The mechanism of action works as follows: the user is trained to keep a posterior pelvic tilt in order to obtain the press of the lumbar curve.

The displacement from this position will produce an alarm sound generated by the acoustic device (5) due to activation of one (1) or both sensors (2), Table 1, Fig 1.

In the bottom part, the device expands with two valves which start from the sacrum, and finish in the middle gluteal. At the end of the two valves, are attached two elastic bands (4), which fix the device to the bottom part of the pelvis, as a security belt, Table 1, Figure 1, Table 2, Fig 3.

## Claims

1. A tool in Celtic cross shape **characterized in that** it causes a proprioceptive action on the person wearing it, that constrains the person to maintain a range of structural alignments that require to be careful with his position in the space, which comprises a rigid structure (6), including two sensors (1) (2) connected to an acoustic device (5), two elastic belts on the dorsal area (3), a belt on the lumbar area (4) Table 1, Fig 1.

2. A tool in Celtic cross shape **characterized in that** it causes a proprioceptive action on the person wearing it, that constrains the person to maintain a range of structural alignments that require to be careful with his position in the space, according to claim 1, in which the sensors (1) and/or (2) activate the acoustic device in case the subject moves away from the correct postural position, Table 1, Fig 1.

3. A tool in Celtic cross shape **characterized in that** it causes a proprioceptive action on the person wearing it, that constrains the person to maintain a range of structural alignments that require to be careful with his position in the space, according to one or more preceding claims, in which the main structure (6) adheres to the person by elastic belts (3) (4), and it forces the subject to maintain a proper posture. Table 1, Fig. 1.
